# FASCICULE DE BREVET EUROPEEN

(11) **EP 1 124 835 B1**
(45) Date de publication et mention de la délivrance du brevet: **02.11.2005**
(21) Numéro de dépôt: 99943019.2
(22) Date de dépôt: 21.09.1999
(51) Int. Cl.: C07H 3/06, C12P 19/14, A01N 63/02, C12N 9/14

(54) **APPLICATION DE FUCO-OLIGOSACCHARIDES SULFATES A LA PROTECTION DES PLANTES**
VERWENDUNGEN VON SULFATIERTEN FUKO-OLIGOSACCHARIDEN ZUM PFLANZENSCHUTZ
USES OF SULFATED FUCO-OLIGOSACCHARIDES FOR PLANT PROTECTION

(30) Priorité: 21.09.1998 FR 9811756
(43) Date de publication de la demande: 22.08.2001
(73) Titulaire: LABORATOIRES GOEMAR S.A., 35400 Saint-Malo (FR)
(72) Inventeur: DESCAMPS, Valérie, F-29680 Roscoff (FR); KLARSZINSKY, Olivier, F-68740 Fessenheim (FR); BARBEYRON, Tristan, F-29233 Cleder (FR); CLOAREC, Bernard, F-29420 Plouenan (FR); FRITIG, Bernard, F-67460 Souffelweyersheim (FR); JOUBERT, Jean-Marie, F-35400 Saint Malo (FR); PLESSE, Bertrand, F-67000 Strasbourg (FR); YVIN, Jean-Claude, F-35400 Saint Malo (FR)
(74) Mandataire: Koch, Gustave
(86) Numéro de dépôt international: PCT/FR1999/002243
(87) Numéro de publication internationale: WO 2000/017215

(56) Documents cités:
- US-A- 5 588 254
- PATENT ABSTRACTS OF JAPAN vol. 95, no. 6, 31 juillet 1995 (1995-07-31) & JP 07 059563 A (TOUSA KOGAKU KENKYUSHO), 7 mars 1995 (1995-03-07) & CHEMICAL ABSTRACTS, vol. 122, no. 21, 22 mai 1995 (1995-05-22) Columbus, Ohio, US; abstract no. 259851,
- PATENT ABSTRACTS OF JAPAN vol. 95, no. 11, 26 décembre 1995 (1995-12-26) & JP 07 215990 A (TOUSA KOGAKU KENKYUSHO), 15 août 1995 (1995-08-15) & DATABASE WPI Week 9541 Derwent Publications Ltd., London, GB; AN 317479

## Description

L'invention décrit des endofucanases propres à la préparation de fuco-oligosaccharides par hydrolyse enzymatique des fucanes.

Elle décrit également une bactérie productrice des endofucanases et la préparation de cette enzyme à partir de la bactérie.

Elle vise, de plus, des gènes d'endofucanases.

Elle a enfin pour objet l'application de ces fuco-oligosaccharides en question à la protection des plantes.

Les fucanes sont des polysaccharides à base de α-L-fucose.

Ils peuvent être extraits de la paroi des algues brunes.

Ils peuvent également être extraits des échinodermes.

On a déjà préparé, par hydrolyse acide ou radicalaire, des fractions oligosaccharidiques à partir des fucanes.

Diverses activités biologiques ont été étudiées tant en rapport avec les fucanes qu'en rapport avec certaines fractions de plus faible poids moléculaire.

On connaît déjà par le Patent Abstracts of Japan, vol. 95, No. 6, 31 juillet 1995 & JP 07 059563 A (TOUSA KOGAKU KENKYUSHO), 7 mars 1995, une enzyme décomposant le fucoïdan purifié et extrait d'un animal de l'espèce Echinoidea.

Le brevet US-A-5 588 254 indique que le produit de décomposition du fucoïdan par un acide ou une enzyme est un oligosaccharide ayant un degré de polymérisation de 2 à 10, cet oligosaccharide ayant la propriété d'accélérer la croissance des plantes.

Le Patent Abstracts of Japan vol. 95, No. 11, 26 décembre 1995 et JP 07 215990 A décrit une composition comprenant un oligosaccharide de fucoïdan de faible poids moléculaire (inférieur à 5000, c'est-à-dire comprenant 26 unités de fucose sulfaté), cet oligosaccharide ayant une excellente solubilité/absorbance dans l'être vivant et conserve les activités biologiques du fucoïdan ; ce fucoïdan de faible poids moléculaire est obtenu par hydrolyse acide du fucoïdan naturel.

L'invention vise des fuco-oligosaccharides particuliers, une enzyme pour leur préparation et leurs applications dans le domaine de la protection des plantes.

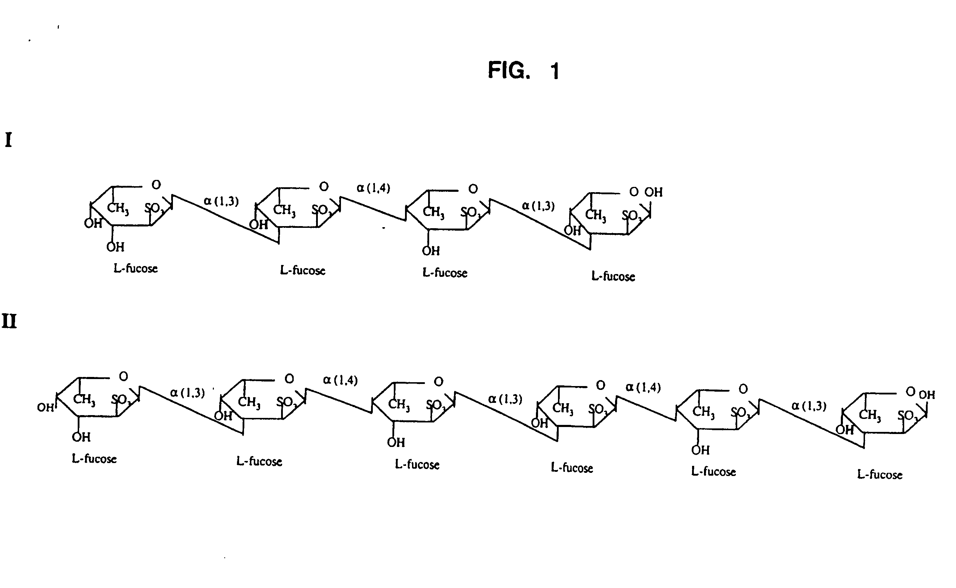

Les fuco-oligosaccharides en question sont sulfatés et ont un degré de polymérisation de 4 à 100, de préférence de 4 à 20 unités α-L fucose; deux d'entre eux sont caractérisés par les formules développées I et II représentées à la figure 1.

Conformément à l'invention, ils sont préparés par hydrolyse enzymatique à partir des fucanes en faisant agir sur ceux-ci des endofucanases, c'est-à-dire des enzymes que la Société Demanderesse a eu le mérite d'isoler.

Ces enzymes sont secrétées par une souche bactérienne à activité fucanolytique que la Société Demanderesse a obtenue à partir des boues industrielles utilisées pour le traitement des effluents, riches en fucanes sulfatés, d'une usine d'extraction d'alginates.

Pour ce faire, elle a procédé comme suit.

Des boues, prélevées dans les bassins d'oxygénation et de décantation d'une station d'épuration située en aval d'une usine d'extraction d'alginates, sont utilisées comme inoculum d'un milieu pauvre à base d'eau de mer filtrée contenant 2 g/l de fucane.

En travaillant sur des parties aliquotes du milieu de culture ainsi constitué, on effectue chaque jour le test dit "à l'alburoine sérique bovine" (ou BSA).

Le principe de ce test est fondé sur le fait que les polysaccharides anioniques s'associent, en milieu acide, avec l'albumine chargée positivement, provoquant ainsi une précipitation de l'ensemble des complexes formés.

Au contraire, les oligosaccharides et les oses ne sont pas précipités ou floculés car ils sont de trop faible poids moléculaire pour donner lieu à la formation d'un complexe insoluble.

En vue du test BSA, on introduit 600 µl du surnageant de culture dans un microtube de 1 ml et on centrifuge.

Le surnageant, à savoir 500 µl, est transféré dans un autre tube et mélangé à 2 ml de solution de BSA acide constituée par 3,26 g d'acétate de sodium, 4,56 ml d'acide acétique glacial et 1 g de BSA dans 1 1 d'eau distillée, le pH étant de 3,72 à 3,78.

On traite parallèlement un témoin qui est constitué par 500 µl de surnageant de culture sans inoculum.

Compte tenu des explications qui précèdent, la formation d'un trouble identique à celui qui se produit dans le témoin indique qu'il n'y a pas eu de dégradation du polymère; par contre, dans le cas où le trouble est moindre ou inexistant, on conclut à la présence, dans la culture bactérienne, d'une activité enzymatique spécifique du fucane.

Le test s'est avéré positif au bout de 7 jours d'incubation du fucane en présence de l'inoculum décrit plus haut.

Une partie aliquote du milieu de culture correspondant est alors étalée sur boîte de Pétri contenant le milieu connu sous l'appellation Zobell-Fucane, qui est constitué par le milieu Zobell (1941) additionné de fucane à 2 g/l et solidifié par de l'agar à 15 g/l.

La composition du milieu Zobell [Zobell CE(1941) "Studies on marine bacteria. I. The cultural requirements of heterotrophic aerobes" paru dans *J*. *Mar. Res.,* 4 : 41-75] est comme suit:

| | |
|---|---|
| Bacto-peptone Difco | 5,0 g |
| Extrait de levure Difco | 1,0 g |
| Eau de mer filtrée | 800 ml |
| Eau distillée q.s.p. | 1 l |

On constate la formation dans la boîte de Pétri d'un certain nombre de colonies différentes les unes des autres et qui correspondent à autant de souches.

Chacune de ces colonies, autrement dit chacune des souches, est isolée à partir de ce milieu et introduite dans un milieu liquide Zobell-Fucane constitué par 1/2 volume de Zobell et 1/2 volume d'une solution de fucane à 4 g/l dans de l'eau de mer filtrée.

Sur chacune des souches ainsi isolées et introduites dans le milieu Zobell-Fucane, on effectue le test BSA pour détecter les souches fucanolytiques.

Lorsque le test est positif, la souche est repiquée sur milieu Zobell-Fucane pour vérifier qu'elle continue à hydrolyser les fucanes du nouveau milieu; si tel est le cas, cela signifie que tout le dispositif génétique nécessaire à la production des fucanases est mis en place; on dit alors que la souche est induite par le substrat; autrement dit, la présence de fucanes dans le milieu induit la production des enzymes par la souche. En raison de cet état de choses, le test BSA est de nouveau effectué sur la nouvelle culture.

En procédant ainsi, on a isolé une souche qui a été dénommée SW5 et qui donne un test BSA positif au bout de 3 à 4 jours lorsqu'elle est cultivée sur milieu Zobell-Fucane.

La susdite souche SW5, sélectionnée de la manière venant d'être décrite, a été déposée sous le n° 12171 dans la collection DSMZ (Deutsche Sammlung von Mikroorganismen und Zellkulturen GmbH) dont l'adresse est la suivante: Mascheroder Weg 1b, D-38124 Braunschweig, Allemagne.

La souche est en bâtonnet Gram négatif, aérobie stricte, chimioorganotrophe et hétérotrophe. Au bout de trois jours de culture, les colonies sur milieu Zobell-Fucane solide sont petites (1 mm de diamètre) et pigmentées en orange. La souche SW5 nécessite de l'eau de mer pour sa croissance, sa température optimale de croissance se situant autour de 18-20°C. Aucune croissance n'est constatée en dessous de 15°C et au-dessus de 25°C. D'autres caractéristiques phénotypiques de la souche SW5 sont montrées dans le tableau I ci-après.

**TABLEAU I**

| **Caractères physiologiques et biologiques de la souche SW5** | | |
|---|---|---|
| Caractères positifs | Caractères négatifs | Caractères négatifs (suite) |
| catalase | oxydase | α-hydroxybutyrate |
| α-cyclodextrine | flexirubine | β-hydroxybutyrate |
| dextrine | réduction des nitrates | γ-hydroxybutyrate |
| glycogène | formation d'indole | p-hydroxyphénylacétate |
| cellobiose | fermentation arginine dihydrol | itaconate |
| D-fructose | uréase | α-kétobutyrate |
| L-fucose | tween 40 | α-kétoglutarate |
| D-galadose | tween 80 | α-kétovalérate |
| gentobiose | N-acétyl-D-galactosamine | malonate |
| α-D-glucose | N-acétyl-D-glucosamine | propionate |
| α-D-lactose | adonitol | quinate |
| maltose | L-arabinose | D-saccharate |
| D-mannose | D-arabitol | sébacate |
| β-méthyl-D-glucoside | érythritol | succinate |
| turanose | m-inositol | bromosuccinate |
| mono-méthyl-succinate | lactulose | succinamate |
| D-galaduronate | D-mannitol | glucuronamate |
| D-glucuronate | D-mélibiose | alininamide |
| D,L-lactate | D-psicose | D-alanine |
| L-alanine | D-raffinose | L-histidine |
| L-alanyl-glycine | L-rhamnose | D,L-carnitine |
| L-asparagine | D-sorbitol | γ-aminobutyrate |
| aspartate | sucrose | inosine |
| L-glutamate | D-tréhalose | uridine |
| glycyl-L-aspartate | xylitol | thymidine |
| glycyl-L-glutamate | méthyl pyruvate | phényléthylamine |
| L-ornithine | acétate | putrescine |
| L-proline | cis-aconitate | 2-aminoéthanol |
| L-thréonine | citrate | 2,3-butanediol |
| urocanate | formate | glycérol |
| glucose-1-phosphate | D-galactanate lactone | D-,L-α-glycérol phosphate |
| glucose-6-phosphate | D-gluconate | |
| | D-glucosaminate | |

Les relations philogénétiques, de la souche SW5 ont été établies par la séquence nucléotidique ID No.1 du gène codant pour l'ARN 16S.

La comparaison de la séquence de ce gène avec les gènes homologues issus de bactéries du groupe CFB (*Cytophaga-Flavobacterium-Bacteroïdes*) a permis de construire un arbre phylogénétique dont il résulte que la souche appartient à la famille des *Flavobacteriaceae* (Bernardet et al, 1996, International Journal of Systematic Bacteriology, Jan: 128-148) et qu'elle est proche de *Gelidibacter algens* (Bowman et al, 1997, International Journal of Systematic Bacteriology, July: 670-677), une souche isolée des glaces de l'Antarctique.

Il s'ensuit que le procédé conforme à l'invention pour la préparation des susdits fuco-oligosaccharides est caractérisé par le fait que l'on soumet un substrat constitué par une matière première à base de fucanes à une hydrolyse enzymatique à l'aide d'une enzyme fucanolytique ou endofucanase susceptible d'être obtenue à partir d'une souche bactérienne déposée sous le N° 12171 dans la collection DSMZ.

La bactérie SW5 secrète dans son milieu de culture des quantités significatives d'activité fucanase, pouvant être utilisées à température ambiante, en milieu tamponné à pH 7,5, voire dans l'eau.

La préparation du substrat, autrement dit des fucanes qui doivent être soumis à l'action de l'activité enzymatique, est effectuée comme suit.

On soumet 150 kg d'algue brune *Pelvetia canaliculata* à un broyage en vue d'obtenir des fragments de 2,5 à 3 mm, en utilisant un dispositif désigné par Comitrol 2100 et commercialisé par la Société Urschel.

Ces fragments sont soumis à une extraction par 800 1 d'acide chlorhydrique 0,01 N contenant 4% en poids de CaCl₂ en maintenant une agitation pendant 3 heures à 70°C.

La suspension de fragments d'algue ainsi traitée est décantée pendant 2 h.30 et le rétentat obtenu est centrifugé; le surnageant de l'opération de centrifugation est soumis à une ultrafiltration tangentielle sur des membranes de 50 kDa. Le rétentat obtenu à l'issue de cette ultrafiltration tangentielle est atomisé à l'aide d'un dispositif désigné par l'appellation Miro et commercialisé par la Société Minor Production.

Le résultat de cette atomisation est une poudre constituée majoritairement par des fucanes.

Cette poudre est soumise à une purification supplémentaire par remise en solution, centrifugation, précipitation du surnageant à l'éthanol, remise en solution du précipité dans l'eau et ultrafiltration tangentielle de la solution aqueuse sur des membranes de 30000 Da.

Le rétentat issu de cette ultrafiltration est soumis à une lyophilisation et constitue le substrat recherché; on le désigne par FS28EtOH.

Ce substrat, autrement dit cette poudre de fucanes, est soumis à l'activité enzymatique obtenue à partir de la bactérie SW5.

Pour ce faire, on prépare une solution à 1 g/l de substrat FS28EtOH dans du tampon Tris (20 mM, pH 7,5); on prélève 50 µl de cette solution et on la mélange à 10 µl de la fraction protéique à tester qui contient l'activité enzymatique obtenue à partir de la bactérie SW5.

Le mélange est maintenu pendant 1 heure à température ambiante.

L'hydrolysat final contient le mélange de fuco-oligosaccharides en question.

Cet hydrolysat est soumis à une électrophorèse en vue de mettre en évidence de façon qualitative la présence de fractions oligosaccharidiques; cette électrophorèse est effectuée sur gel de polyacrylamide en utilisant la technique dite C-PAGE décrite par Zablackis E. et Perez J. (1990) dans l'article intitulé "A partially pyruvated carrageenan from Hawaiian *Grateloupia filicina* (Crypto-nemiales, Rhodophyta) paru dans *"Bot. Mar*." 33: 273-276.

Les profils de dégradation des fucanes obtenus à l'aide de l'enzyme produite par la bactérie SW5 montrent que l'activité enzymatique de la souche en question est de type endolytique; il a été remarqué au cours des essais que les fucanes présentent des éléments de structure semblables quelle que soit l'algue brune dont ils sont extraits.

L'activité endo-fucanase de la souche SW5 a été purifiée à l'homogénéité électrophorétique; on a déterminé que la protéine en question a une masse moléculaire d'environ 105 kDa.

Trois peptides internes ont alors été micro-séquencés.

On a ainsi obtenu les microséquences ID No.2, ID No.3 et ID No.4.

L'invention vise également les endofucanases caractérisées par le fait qu'ils contiennent des séquences analogues aux peptides ID No.2, ID No.3, ID No. 4 et/ou ID No. 6 ainsi que les gènes d'endofucanase caractérisés par le fait qu'ils contiennent des séquences analogues à la séquence nucléotidique ID No. 5.

La purification de l'activité endo-fucanase dont il a été question plus haut, a été effectuée à l'aide d'un système commercialisé sous l'appellation FPLC® par la Société Pharmacia; ce système a été placé à 4°C.

Après cinq jours dé culture de la souche SW5 dans 6 1 de milieu Zobell-Fucane, on procède à une micro-filtration sur un dispositif du type Pellicon de la Société Millipore équipé d'une cassette de 0,45 µm (Millipore) puis à une ultrafiltration tangentielle sur une cassette PTGC de 30 000 Da.

Pour le fractionnement des protéines, on procède de la façon suivante:

Pour le premier fractionnement de 0 à 40%, on ajoute à la solution protéique du (NH₄)₂SO₄ à 242 g/l petit à petit et sous agitation lente à 4°C. Lorsque tout le sulfate d'ammonium est dissous, la solution est centrifugée; le culot, qui représente la fraction entre 0 et 40% n'est pas conservé tandis que le surnageant est soumis à un nouveau fractionnement de 40 à 60%. Ce fractionnement est réalisé par addition de (NH₄)₂SO₄ à 130 g/l petit à petit et sous agitation lente à 4°C. Lorsque les cristaux sont dissous, la solution est centrifugée; le culot, qui représente la fraction protéique entre 40 et 60% de saturation du sulfate d'ammonium, est conservé et resolubilisé dans le tampon Tris 20 mM pH 7,5, NaCl 50 mM, CaCl₂ 5 mM, MgCl₂ 5 mM.

La fraction entre 40 et 60% de saturation du sulfate d'ammonium est diluée 20 fois dans du tampon Tris 20 mM pH 7,5 et resaturée à 40% de (NH₄)₂SO₄.

L'échantillon ainsi obtenu est injecté sur une colonne de chromatographie d'interaction hydrophobe à base de Phenyl Sepharose CL4B Pharmacia à raison de 1 ml/min.

La colonne est préalablement équilibrée avec du tampon Tris pH 7,5; (NH₄)₂SO₄ à 40%. Le gradient s'effectue par diminution de la concentration en (NH₄)₂SO₄ de 1,8 M (40%) à 0 M dans du tampon Tris en 3 volumes de colonne, puis par 2 volumes de tampon sans (NH₄)₂SO₄.

Chaque fraction protéique est utilisée pour hydrolyser le fucane FS28EtOH. L'hydrolysat est préparé comme décrit précédemment par ajout de ladite fraction à une solution de fucane à 1 g/l dans du tampon Tris 20 mM pH 7,5 contenant des sels. L'hydrolysat est alors testé par la technique C-PAGE. Les fractions dites "positives sur C-PAGE" sont celles qui génèrent un profil électrophorétique contenant des fractions d'oligo-fucosaccharides.

Les fractions qui génèrent un profil de dégradation positif sur gel de polyacrylamide sont rassemblées et diluées 5 fois dans du tampon Tris 20 mM pH 7,5; NaCl 5 mM avant d'être injectées sur une colonne de chromatographie d'échange d'anions chargée en DEAE Sepharose CL6B commercialisé par la Société Pharmacia.

Les protéines sont éluées par un gradient de NaCl de 5 mM à 1 M en 3 volumes de colonne, à 1 ml/min.

Ces fractions sont ensuite injectées à raison de 100 µl sur une colonne de filtration, chargée de gel Superdex 200 commercialisé par la Société Pharmacia et préalablement équilibrée à raison de 0,5 ml/min dans du tampon phosphate de sodium 50 mM pH 7,0; NaCl 150 mM.

La fraction active, identifiée par la technique C-PAGE, est recueillie et la pureté de la protéine est déterminée sur un gel SDS à 12,5% en utilisant le dispositif Phast-System® de la Société Pharmacia. Les marqueurs moléculaires utilisés sont les standards de 14, 5 à 200 kDa commercialisés sous l'appellation « broad-range » par la Société Biorad.

A partir des microséquences protéiques SEQ ID N° 3 et SEQ ID N° 4, on a synthétisé des oligonucléotides ; on a utilisé ces oligonucléotides comme amorces afin de synthétiser par PCR (Polymerase Chain Reaction) un fragment du gène codant pour la fucanase, l'ADN purifié de la souche SW5 servant de matrice à la réaction.

Les produits de PCR spécifiques des couples d'amorces ont été séquencés.

L'un d'entre eux présente la séquence nucléotidique SEQ ID N° 5.

La séquence protéique déduite, longue de 67 acides aminés, contient les microséquences SEQ ID N°3 et SEQ ID N°4, indiquant qu'il s'agit d'un fragment de la fucanase de la souche SW5.

La séquence nucléotidique SEQ ID N° 5 est donc une portion du gène, longue de 203 nucléotides, qui code pour la fucanase de la souche SW5.

Pour la préparation des fuco-oligosaccharides, on peut avoir recours à plusieurs méthodes dont deux sont décrites ci-après.

Dans une première méthode, on laisse agir l'enzyme jusqu'à consommation complète du substrat; on incube pendant 24 heures un fucoïdane de *Pelvetia canaliculata* en présence de l'enzyme partiellement purifiée, puis on ultrafiltre les produits d'hydrolyse sur membrane de 500 Da. On obtient ainsi une fraction désignée par 127.

Du point de vue pratique, on hydrolyse un fucane extrait de *Pelvetia canaliculata* (fraction FS28EtOH) à la température ambiante pendant 24 heures, en ajoutant à 1 litre de substrat d'une concentration de 5 g/l dans le tampon Tris 20 mM pH 7,5; NaCl 50 mM; MgCl₂ 5 mM; CaCl₂ 5 mM, une quantité de 30 ml de précipité d'enzyme au sulfate d'ammonium 40-60% (3,02 mg de protéines).

L'hydrolysat ainsi obtenu est complété par de l'eau distillée jusqu'à 20 1, puis ultrafiltré sur un système d'ultrafiltration tangentielle de 10 000 Da en utilisant une cassette 0,46 m² PTGC de la Société Millipore avec une pression d'entrée de 6,5 bars et une pression de sortie de 5,5 bars.

L'ultrafiltrat ainsi obtenu est récupéré et de nouveau ultrafiltré sur 500 Da en utilisant le dispositif commercialisé sous la marque Omega Centraset OM500DC05 par la Société Pall Filtron avec 1 bar en entrée de circuit et une pression nulle de sortie; cette nouvelle ultrafiltration tangentielle vise à dessaler l'ultrafiltrat.

En vue de fractionner les oligomères, le rétentat est de nouveau ultrafiltré sur 500 Da mais avec des pressions d'entrée de 2,5 bars et 2 bars à la sortie.

Le filtrat qui passe le seuil de coupure de 500 Da (environ 20 1) de la membrane est ensuite concentré sur évaporateur rotatif, neutralisé au carbonate d'ammonium puis lyophilisé en utilisant le dispositif commercialisé sous la marque Lyolab A LSL par la Société Secfroid. Le lyophilisat ainsi obtenu contient les oligofucanes appelés I27.

Les oligofucanes de la fraction I27 ainsi obtenus ont été soumis à un fractionnement supplémentaire par chromatographie d'échange d'ions et chromatographie d'exclusion.

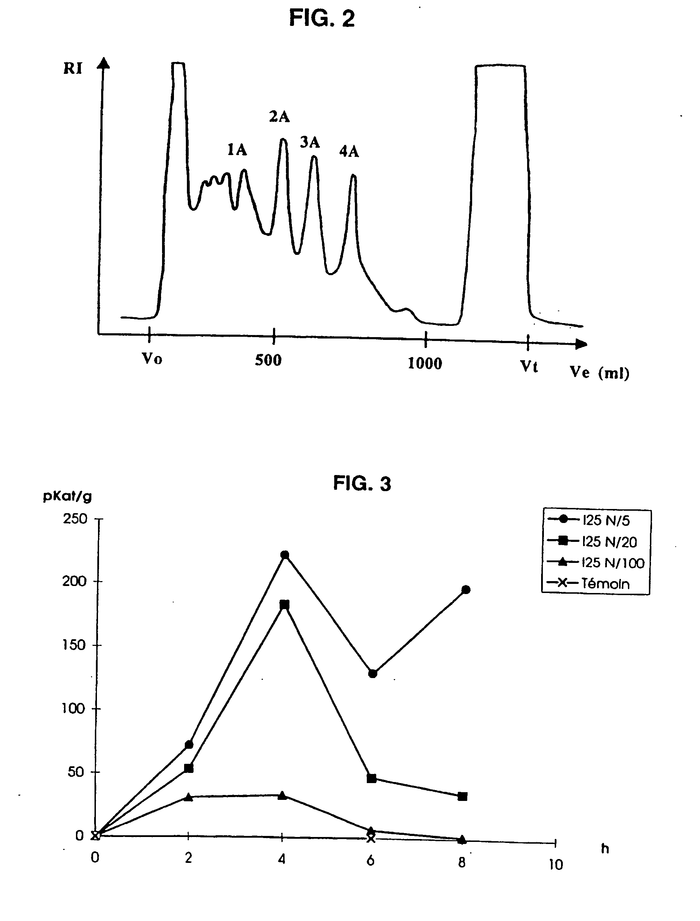

Le chromatogramme d'exclusion obtenu sur un gel de la marque BIOGEL P6 est reproduit sur le graphique de la figure 2 qui montre en abscisse le volume d'élution (Ve) exprimé en ml et en ordonnées l'indice réfractométrique (RI) de l'éluat.

La structure des oligosaccharides ainsi obtenus (pics 1A à 4A du chromatogramme) a été analysée par RMN bidimensionnelle.

Il s'agit, dans le cas des pics 4A et 3A, respectivement d'un tétrasaccharide et d'un hexasaccharide formés par des résidus de fucose alternativement reliés par des liaisons α(1->3) et α(1->4) et sulfatés sur leur carbone 2 (figure 1).

Les pics 2A et 1A sont, quant à eux, constitués par des mélanges de DP variable d'oligosaccharides de la même série homologue, mais pouvant comporter des branchements.

Ce résultat suggère que les polysaccharides de départ sont constitués, au moins partiellement, par la répétition de motifs α-L-Fuc-2-sulfate-1->3-α-L-Fuc-2-sulfate-1->4 non décrits à ce jour.

Une deuxième méthode comporte une hydrolyse en continu; elle consiste, à l'aide d'une membrane d'ultrafiltration de 30 kDa, à soustraire à l'action de l'enzyme les oligofucanes à mesure de leur formation. La fraction ainsi obtenue est désignée par "fraction I25" et contient des oligofucanes de masse moléculaire plus élevée que ceux constituant la fraction I27.

Du point de vue pratique, on dissout le substrat (50 g de FS28EtOH) dans 10 litres d'eau distillée.

Pour effectuer l'hydrolyse, on ajoute 25 ml d'enzyme sous forme d'une fraction précipitée au sulfate d'ammonium entre 40 et 60% de saturation, dans 2 litres de substrat; l'ultrafiltration en continu est engagée après 30 minutes.

Pour cette ultrafiltration tangentielle, on utilise un appareil de marque Pellicon comportant une cassette de 30 000 Da, ledit appareil étant réglé sur 1 bar en entrée et 0 bar en sortie pendant toute la durée de l'hydrolyse.

La sortie du filtrat est partiellement fermée pour maintenir le débit de filtration à 2 1/h.

Les caractéristiques de l'enceinte réactionnelle sont choisies de façon à permettre un approvisionnement de l'enzyme en substrat jusqu'à épuisement des 10 litres et le maintien d'un volume fixe d'hydrolyse de 2 litres.

L'hydrolyse est poursuivie en ajoutant de l'eau distillée (8 litres) en continu jusqu'à ce que le filtrat ne contienne plus d'oligomères.

On obtient 18 litres d'un ultrafiltrat qui est concentré jusqu'à environ 1 litre à l'aide d'un appareil Pellicon équipé d'une cassette de 500 Da, la pression d'entrée étant de 2 bars et la pression de sortie de 0; une concentration supplémentaire jusqu'à 200 ml est effectuée par évaporation rotative, puis le concentrat est neutralisé et lyophilisé.

Le filtrat, qui contient les fractions de faible poids moléculaire (c'est-à-dire capable de passer sur une membrane au seuil de coupure de 500 Da) n'a pas été conservé.

La fraction I25 désigne les oligofucanes retenus entre les seuils de coupure de 500 Da et 30000 Da.

On a étudié l'activité biologique des fractions I27 et I25 sur les plantes.

L'effet éliciteur direct de ces fractions a été analysé sur des cultures de cellules de Tabac BY.

Cinq marqueurs des réactions de défense ont été testés indépendamment, à savoir:
- l'alcalinisation du milieu de culture (réponse précoce couramment observée lorsque les plantes sont incubées en présence d'éliciteurs oligosaccharidiques),
- les niveaux d'activité "phénylammonia lyase" (PAL) qui est une enzyme clé pour la synthèse des phytoalexines chez les plantes,
- les niveaux d'activité O-méthyl transférase (OMT) qui est une enzyme impliquée dans la synthèse de lignine,
- les niveaux d'activité lipoxygénase (LOX) qui est une enzyme impliquée dans la génération de jasmonate de méthyle, un élément des cascades de signalisation aboutissant à l'activation de gènes de défense et
- les teneurs en acide salicylique (SA) qui est un autre messager secondaire impliqué dans les réactions de défense.

La fraction 127, administrée à une dose de 200 mg/l, induit une alcalinisation du milieu de culture des cellules de tabac de 1 à 1,5 unités pH. Par rapport aux cellules non traitées, les activités PAL et OMT sont stimulées respectivement d'un facteur 50 à 600 et 2,0 à 2,8, entre 4 heures et 8 heures après l'addition de I27 au milieu de culture.

L'activité LOX, mesurée 18 heures après l'addition de I27 à la dose de 200 mg/l est 7 fois plus importante que dans les cellules témoins. De même, 3 heures après le début du traitement, les teneurs en acide salicylique sont 70 fois plus importantes que dans les cellules non traitées.

Des résultats analogues ont été observés avec la fraction I25. On note toutefois une stimulation plus forte de l'activité LOX, jusqu'à un facteur multiplicatif de 55 par rapport aux cellules témoins.

La figure 3 illustre l'influence de la fraction I25 sur la stimulation de l'activité PAL dans les cellules de tabac BY; elle montre la variation de l'activité exprimée en pico-katal par g (pKat/g) de masse fraîche de suspension cellulaire en fonction du temps exprimé en heures pour trois concentrations, à savoir 200 mg/l, 50 mg/l et 10 mg/l et pour un témoin.

L'examen de la figure 3 montre que des concentrations de 10 mg/l sont suffisantes pour déceler une stimulation significative par rapport aux cellules témoins non élicitées et on observe une saturation de la réponse pour les concentrations supérieures à 50 mg/l.

Les résultats de ces expériences montrent que les oligofucanes conformes à l'invention sont reconnus comme des éliciteurs par les cellules de tabac.

Par rapport à des cellules non traitées, ils stimulent très fortement (c'est-à-dire dans des proportions tout à fait comparables à celles d'autres éliciteurs comme les oligopectines et les oligo β1-3 glucanes), les activités PAL, LOX et OMT ainsi que les teneurs en SA.

L'effet éliciteur de la fraction I25 a également été analysé sur des suspensions cellulaires de blé.

On montre que cette fraction induit l'activité PAL.

Ainsi, à la concentration de 200 mg/l, I25 a stimulé d'un facteur 15 à 20 l'activité PAL entre 2 et 6 heures après le traitement.

Une dose de 50 mg/l stimule encore l'activité PAL d'un facteur 4, 4 heures après le traitement.

I25 induit également l'activité PAL dans les suspensions cellulaires de persil.

A 200 mg/l I25 a stimulé d'un facteur 15 à 30 l'activité PAL entre 4 et 6 heures après le traitement.

Une dose de 20 mg/l stimule encore l'activité PAL d'un facteur 5, 4 heures après le traitement.

Par une autre série d'expériences, on montre que, infiltrés dans les feuilles de tabac, les fuco-oligosaccharides protègent contre le VMT (virus de la mosaïque du tabac). Les expériences ont été réalisées comme suit.

La fraction de fuco-oligosaccharides dénommée I25 (200 mg/l) est infiltrée à l'aide d'une seringue dans trois feuilles de tabac (feuilles 4, 5 et 6 en partant du bas) de 2 mois d'âge.

Cinq jours après l'infiltration, une inoculation au VMT est réalisée dans les feuilles 5, 6 et 7, la feuille 4 servant de témoin.

Une semaine après l'inoculation, on détermine le nombre de lésions (NL) et la taille de ces lésions (TL) sur les feuilles des plants traitées ainsi que sur des plants traités à l'eau uniquement.

Dans le tableau II ci-après sont représentés les pourcentages de protection, selon les deux critères définis ci-dessus.

**TABLEAU II**

| Protection du tabac contre le VMT par les fuco-oligosaccharides | | |
|---|---|---|
| | % protection (NL) | % protection (TL) |
| Feuille 5 | 65 | 25 |
| Feuille 6 | 65 | 35 |
| Feuille 7 | 40 | 26 |

La feuille 4 ne présente aucune lésion.

Des résultats réunis dans ce tableau, on peut conclure que l'on a bien induit une protection dans les feuilles infiltrées par I25 (feuilles 5 et 6), mais aussi dans la feuille 7, qui n'avait pas reçu de fuco-oligosaccharides.

On peut donc parler de protection systémique.

De ces deux séries d'expériences sur le blé, le persil et le tabac, il ressort que les fuco-oligosaccharides stimulent des activités enzymatiques marqueurs des défenses naturelles non seulement dans le cas du tabac, mais également dans le cas du blé et du persil.

Cette stimulation aboutit à une protection systémique contre le VMT et, vraisemblablement, contre d'autres agents phytopathogènes.

A titre illustratif, on donne ci-après deux exemples.

### EXEMPLE 1

Concentré liquide pour l'agriculture à base d'oligofucanes:

| | |
|---|---|
| Oligofucane I25 | 0,200 kg |
| Tween 801 | 0,005 kg |
| Méthylparaben sodé | 0,005 kg |
| Eau | 0,790 kg |
| Total | 1,000 kg |

Ce concentré est utilisé après dilution dans 1000 litres d'eau d'une quantité comprise entre 10 g et 10 kg, de préférence entre 20 g et 5 kg et plus préférentiellement entre 100 g et 1 kg ; cette dilution présente une teneur en oligofucanes I25 comprise entre 2 et 2000 g, de préférence entre 20 et 200 g pour 1000 litres d'eau.

### EXEMPLE 2

Poudre concentrée soluble contenant, en tant que matière active, l'hydrolysat I27 ainsi que des adjuvants.

Pour 1 kg en poids/poids, la constitution de cette poudre est définie comme suit :

| | |
|---|---|
| Oligofucane I27 | 0,200 kg |
| Kaolin | 0,495 kg |
| Mannose | 0,050 kg |
| Méthylparaben sodé | 0,005 kg |
| Amidon purifié | 0,250 kg |
| Total | 1,000 kg |

Cette poudre est utilisée après dilution dans une quantité d'eau suffisante pour obtenir une composition dont la teneur en oligofucane I27 est comprise entre 2 et 5000 g, de préférence entre 20 g et 100 g pour 1000 litres d'eau.

### LISTE DE SEQUENCES

(1) INFORMATIONS GENERALES:
   (i) DEPOSANT:
      (A) NOM: Laboratoires GOËMAR S.A.
      (B) RUE: ZAC de la Madeleine
      (C) VILLE: SAINT MALO
      (E) PAYS: FRANCE
      (F) CODE POSTAL: 35400
      (G) TELEPHONE: +33 2 99 21 53 70
      (G) TELEFAX: +33 2 99 8256 17
      (G) TELEX:
   (ii) TITRE DE L'INVENTION: Fuco-oligosaccharides, enzyme pour leur préparation à partir des fucanes, bactérie productrice de l'enzyme et applications des fuco-oligosaccharides à la protection des plantes.
   (iii) NOMBRE DE SEQUENCES: 4
   (iv) FORME DECHIFFRABLE PAR ORDINATEUR:
      (A) TYPE DE SUPPORT: disquette
      (B) ORDINATEUR: IBM PC compatible
      (C) SYSTEME D' EXPLOITATION: PC-DOS/MS-DOS
      (D) LOGICIEL:
   (v) DONNEES DE LA DEMANDE ACTUELLE:
      NUMERO DE LA DEMANDE:
      DATE DE DEPOT: 21 septembre 1998
(2) INFORMATIONS POUR LA SEQ ID NO: 1:
   (i) CARACTERISTIQUES DE LA SEQUENCE:
      (A) LONGUEUR: 1474 nucléotides
      (B) TYPE: séquence nucléotidique
      (C) NOMBRE DE BRINS: double brin
      (D) CONFIGURATION: linéaire
   (ii) TYPE DE MOLECULE: gène de l'ARN 16S
   (iii) HYPOTHETIQUE: non
   (iv) ANTI-SENS: non
   (vi) ORIGINE:
      (A) ORGANISME: CNRS, Station Biologique, Laboratoire de Biologie cellulaire et moléculaire des algues, 29682 Roscoff Cedex
      (B) SOUCHE: SW5
   (xi) DESCRIPTION DE LA SEQUENCE: SEQ ID NO: 1:
   AGAGTTTGAT CNTGGCTCAG GATGAACGCT AGCGGCAGGC CTAACACATG CAAGTCGAGG   060
   GGTAGAGAGA GCTTGCTTTT CTTGAGACCG GCGCACGGGT GCGTAACGCG TATACAATCT   120
   GCCTCTTACT GCGGGATAGC CCAGAGAAAT TTGGATTAAT ATCGCATAGC ATAACGACCC   180
   CGCATGGGAT GTTATTAAAG GTTACGGTAA GAGATGAGTA TGCGTTCTAT TAGCTAGATG   240
   GAGTGGTAAC GGCACACCAT GGCAACGATA GATAGGGGCC CTGAGAGGGG GATCCCCCAC   300
   ACTGGTACTG AGACACGGAC CAGACTCCTA CGGGAGGCAG CAGTGAGGAA TATTGGACAA   360
   TGGAGGCAAC TCTGATCCAG CCATGCCGCG TGCAGGAAGA CGGCCCTATG GGTTGTAAAC   420
   TGCTTTTATA CGGGAAGAAA CACCGCTACG TGTAGCCTTT GACGGTACCG TAAGAATAAG   480
   GATCGGCTAA CTCCGTGCCA GCAGCCGCGG TAATACGGAG GATCCAAGCG TTATCCGGAA   540
   TCATTGGGTT TAAAGGGTCC GTAGTGGATG ATTAAGTCAG AGGTGAAATC CTGCCGCTCA   600
   ACGGTAGAAT TGCCTTTGAT ACTGGTTATC TTGAATCAAT GTGAAGTGGT TAGAATATGT   660
   AGTGTAGCGG TGAAATGCAT AGATATTACA TAGAATACCA ATTGCGAAGG CAGATCACTA   720
   ACATTGTATT GACACTGATG GACGAAAGCG TGGGGAGCGA ACAGGATTAG ATACCCTGGT   780
   AGTCCACGCC GTAAACGATG GATACTAGCT GTTCGGATTT ATCTGAGTGG CTAAGCGAAA   840
   GTGATAAGTA TCCCACCTGG GGAGTACGTT CGCAAGAATG AAACTCAAAG GAATTGACGG   900
   GGGCCCGCAC AAGCGGTGGA GCATGTGGTT TAATTCGATG ATACGCGAGG AACCTTACCA   960
   GGGCTTAAAT GTAGATTGCA TTAGGTGGAG ACACTTATTT CTTCGGACCA TCTACAAGGT  1020
   GCTGCATGGT TGTCGTCAGC TCGTGCCGTG AGGTGTCAGG TTAAGTCCTA TAACGAGCGC  1080
   AACCCCCGTT GTTAGTTGCC AGCGAGTCAT GTCGGGAACT CTANCAAGAC TGCCAGTGCA  1140
   AACTGTGAGG AAGGTGGGGA TGACGTCAAA TCATCACGGC CCTTACGTCC TGGGCTACAC  1200
   ACGTGCTACA ATGGTAGGGA CAGAGAGCAG CCACTGGGCG ACCAGGAGCG AATCTATAAA  1260
   CCCTATCACA GTTCGGATCG GAGTCTGCAA CTCGACTCCG TGAAGCTGGA ATCGCTAGTA  1320
   ATCGCATATC AGCCATGATG CGGTGAATAC GTTCCCGGGC CTTGTACACA CCGCCCGTCA  1380
   AGCCATGGAA GCTGGGAGTG CCTGAAGTCC GTCACCGCAA GGAGCGGCCT AGGGTAAAAT  1440
   CGGTAACTAG GGCTAAGTCG TAACAAGGTG TCCG                              1474
(3) INFORMATIONS POUR LA SEQ ID NO: 2:
   (i) CARACTERISTIQUES DE LA SEQUENCE:
      (A) LONGUEUR: 15 acides aminés
      (B) TYPE: séquence peptidique
      (C) NOMBRE DE BRINS: simple brin
      (D) CONFIGURATION: linéaire
   (ii) TYPE DE MOLECULE: protéine
   (iii) HYPOTHETIQUE: non
   (iv) ANTI-SENS: non
   (vi) ORIGINE:
      (A) ORGANISME: CNRS, Station biologique, Laboratoire de biologie cellulaire et moléculaire des algues, 29682 Roscoff Cedex
      (B) SOUCHE: SW5
   (xi) DESCRIPTION DE LA SEQUENCE: SEQ ID NO: 2:
   Gln Thr Ala Asn Thr Thr Tyr Gly Ile Asn Thr Val Ala Ser Met
                    5                  10                  15
(4) INFORMATIONS POUR LA SEQ ID NO: 3:
   (i) CARACTERISTIQUES DE LA SEQUENCE:
      (A) LONGUEUR: 18 acides aminés
      (B) TYPE: séquence peptidique
      (C) NOMBRE DE BRINS: simple brin
      (D) CONFIGURATION: linéaire
   (ii) TYPE DE MOLECULE: protéine
   (xi) DESCRIPTION DE LA SEQUENCE: SEQ ID NO: 3:
   Thr Ser Gly Pro Asp Trp Leu Thr Ile Gln Gln Thr Asp Ala Asn Ser
                   5                   10                  15
   Gly Lys
(5) INFORMATIONS POUR LA SEQ ID NO: 4:
   (i) CARACTERISTIQUES DE LA SEQUENCE:
      (A) LONGUEUR: 17 acides aminés
      (B) TYPE: séquence peptidique
      (C) NOMBRE DE BRINS: simple brin
      (D) CONFIGURATION: linéaire
   (ii) TYPE DE MOLECULE: protéine
   (xi) DESCRIPTION DE LA SEQUENCE: SEQ ID NO: 4:
   Ile Thr Val Asp His Val Ala Gly Phe Thr Asn Leu Trp Asn Gly Ala
                   5                   10                  15
   Pro
(6) INFORMATIONS POUR LA SEQ ID NO: 5:
   (i) CARACTERISTIQUES DE LA SEQUENCE:
      (A) LONGUEUR: 203 paires de bases
      (B) TYPE: séquence nucléotidique
      (C) NOMBRE DE BRINS: double brin
      (D) CONFIGURATION: linéaire
   (ii) TYPE DE MOLECULE: gène partiel de la fucanase
   (iii) HYPOTHETIQUE: non
   (iv) ANTI-SENS: non
   (vi) ORIGINE:
      (A) ORGANISME: CNRS, Station Biologique, Laboratoire de Biologie cellulaire et moléculaire des algues, 29682 Roscoff Cedex
      (B) SOUCHE: SW5
   (xi) DESCRIPTION DE LA SEQUENCE: SEQ ID NO: 5:
   ATTACGGTTG ATCATGTTGC AGGTTTTACT AATTTGGGTA ATGGAGCACC TGTTTGGTCT   060
   TCACCTATAC TTAATCTTAC CGATGGAAAA GGATCATTCG CCTATAATTA TACTTTGCAA   120
   TTAGGAACCG ATTATTATGA TTTTGAAGGT GATGCACTTA CTATTACTAA AACATCAGGA   180
   CCTGATTGGC TCACCATTCA ACA                                           203
(7) INFORMATIONS POUR LA SEQ ID NO: 6:
   (i) CARACTERISTIQUES DE LA SEQUENCE:
      (A) LONGUEUR: 67 acides aminés
      (B) TYPE: séquence protéique
      (C) NOMBRE DE BRINS: simple brin
      (D) CONFIGURATION: linéaire
   (ii) TYPE DE MOLECULE: protéine
   (iii) HYPOTHETIQUE: non
   (iv) ANTI-SENS: non
   (vi) ORIGINE:
      (A) ORGANISME: CNRS, Station Biologique, Laboratoire de Biologie cellulaire et moléculaire des algues, 29682 Roscoff Cedex
      (B) SOUCHE: SW5
   (xi) DESCRIPTION DE LA SEQUENCE: SEQ ID NO: 6:
   Ile Thr Val Asp His Val Ala Gly Phe Thr Asn Leu Gly Asn Gly   15
   Ala Pro Val Trp Ser Ser Pro Ile Leu Asn Leu Thr Asp Gly Lys   30
   Gly Ser Phe Ala Tyr Asn Tyr Thr Leu Gln Leu Gly Thr Asp Tyr   45
   Tyr Asp Phe Glu Gly Asp Ala Leu Thr Ile Thr Lys Thr Ser Gly   60
   Pro Asp Trp Leu Thr Ile Gln                                   67

## Revendications

1. Utilisation pour un traitement de protection des plantes, de fuco-oligosaccharides sulfatés, dont le degré de polymérisation est de 4 à 100, de préférence de 4 à 20 unités α-L-fucose, sulfatés sur leur carbone 2.

2. Utilisation selon la revendication 1, **caractérisée par le fait que** le fuco-oligosaccharide utilisé est un tétrasaccharide formé par des résidus de fucose sulfatés alternativement reliés par les liaisons α(1-3) et α(1→4).

3. Utilisation selon la revendication 1, **caractérisée par le fait que** le fuco-oligosaccharide utilisé est un hexasaccharide formé par des résidus de fucose sulfatés alternativement reliés par les liaisons α(1→3) et α(1→4).

4. Utilisation pour un traitement de protection des plantes d'une composition phytopharmaceutique comportant des fuco-oligosaccharides sulfatés dont le degré de polymérisation est de 4 à 100, de préférence de 4 à 20 unités α-L-fucose, sulfatés sur leur carbone 2.

5. Utilisation selon la revendication 4, **caractérisée par le fait que** le fuco-oligosaccharide comporté par la composition phytopharmaceutique est un tétrasaccharide formé par des résidus de fucose sulfatés alternativement reliés par les liaisons α(1→3) et α(1→4).

6. Utilisation selon la revendication 4, **caractérisée par le fait que** le fuco-oligosaccharide utilisé est un hexasaccharide formé par des résidus de fucose sulfatés alternativement reliés par les liaisons α(1→3) et α(1→4).

## Patentansprüche

1. Verwendung von sulfatierten Fuco-Oligosacchariden für eine Pflanzenschutz-Behandlung, wobei der Polymerisationsgrad der sulfatierten Fuco-Oligosaccharide 4 bis 100, bevorzugt 4 bis 20, α-L-Fucose-Einheiten, die auf ihrem Kohlenstoff 2 sulfatiert sind, beträgt.

2. Verwendung gemäß Anspruch 1, **dadurch gekennzeichnet, dass** das verwendete Fuco-Oligosaccharid ein Tetrasaccharid ist, das durch sulfatierte Fucosereste gebildet wird, die abwechselnd durch α(1→3)- und α(1→4)-Bindungen verbunden sind.

3. Verwendung gemäß Anspruch 1, **dadurch gekennzeichnet, dass** das verwendete Fuco-Oligosaccharid ein Hexasaccharid ist, das durch sulfatierte Fucosereste gebildet wird, die abwechselnd durch α(1→3)- und α(1→4)-Bindungen verbunden sind.

4. Verwendung einer phytopharmazeutischen Zusammensetzung für eine Pflanzenschutz-Behandlung, wobei die Zusammensetzung sulfatierte Fuco-Oligosaccharide enthält, deren Polymerisationsgrad 4 bis 100, bevorzugt 4 bis 20, α-L-Fucose-Einheiten, die auf ihrem Kohlenstoff 2 sulfatiert sind, beträgt.

5. Verwendung gemäß Anspruch 4, **dadurch gekennzeichnet, dass** das in der phytopharmazeutischen Zusammensetzung enthaltene Fuco-Oligosaccharid ein Tetrasaccharid ist, das durch sulfatierte Fucosereste gebildet wird, die abwechselnd durch α(1→3)- und α(1→4)-Bindungen verbunden sind.

6. Verwendung gemäß Anspruch 4**, dadurch gekennzeichnet, dass** das verwendete Fuco-Oligosaccharid ein Hexasaccharid ist, das durch sulfatierte Fucosereste gebildet wird, die abwechselnd durch α(1→3)- und α(1→4)-Bindungen verbunden sind.

## Claims

1. Use for a treatment of protection of plants, of sulphated fuco-oligosacharides, whose degree of polymerisation is from 4 to 100, preferably from 4 to 20 α-L fucose units, sulfated on their carbon 2.

2. Use according to claim 1, **characterized by** the fact that the fuco-oligosaccharide which is used is a tetrasaccharide constituted by sulfated fucose residue alternatively linked by α(1→3) and α(1→4) links.

3. Use according to claim 1, **characterized by** the fact that the fuco-oligosaccharide which is used is a hexasaccharide constituted by sulfated fucose residue alternatively linked by α(1→3) and α(1→4) links.

4. Use for a treatment of protection of plants, of a phytopharmaceutical composition comprising sulphated fuco-oligosacharides, whose degree of polymerisation is from 4 to 100, preferably from 4 to 20 α-L fucose units, sulfated on their carbon 2.

5. Use according to claim 4, **characterized by** the fact that the fuco-oligosaccharide comprised by the phytopharmaceutical composition is a tetrasaccharide constituted by sulfated fucose residue alternatively linked by α(1→3) and α(1→4) links.

6. Use according to claim 4, **characterized by** the fact that the fuco-oligosaccharide which is used is a hexasaccharide constituted by sulfated fucose residue alternatively linked by α(1→3) and α(1→4) links.
